# EUROPEAN PATENT SPECIFICATION

(11) **EP 3 008 162 B1**
(45) Date of publication and mention of the grant of the patent: **31.05.2017**
(21) Application number: 14728812.0
(22) Date of filing: 06.05.2014
(51) Int. Cl.: C12M 1/00, C12N 1/02

(54) **METHOD FOR SEPARATION OF SPORADIC CELLS FROM BODY FLUIDS, AND APPARATUS FOR CARRYING OUT SAID METHOD**
VERFAHREN ZUR TRENNUNG SPORADISCHER ZELLEN VON KÖRPERFLÜSSIGKEITEN SOWIE VORRICHTUNG ZUR DURCHFÜHRUNG DES VERFAHRENS
PROCEDE POUR LA SEPARATION DE CELLULES SPORADIQUES A PARTIR DE LIQUIDES ORGANIQUES ET APPAREIL POUR LA MISE EN OEUVRE DUDIT PROCEDE

(30) Priority: 14.06.2013 CZ 20130456
(43) Date of publication of application: 20.04.2016
(73) Proprietor: Metacell, S.r.o., 70300 Ostrava (CZ)
(72) Inventor: BOBEK, Vladimir, 10300 Praha 3 (CZ); KOLOSTOVA, Katarina, 94911 Nitra (SK)
(74) Representative: Korejzová, Petra
(86) International application number: PCT/CZ2014/000052
(87) International publication number: WO 2014/198242

(56) References cited:
- EP-A2- 2 634 245
- WO-A1-2006/116327
- WO-A2-2012/057498
- US-A- 5 114 350
- US-A1- 2010 324 449

## Description

### FIELD OF THE INVENTION

The present invention provides a method of gentle separation of viable sporadic cells (rare cells) from body fluids such as blood, from malignant effusions (ascites, pleural effusion), bronchoalveolar lavage fluid, peritoneal lavage fluid and amniotic fluid. Using the present method it is possible to isolate for example circulating (CTCs, circulating tumor cells) and disseminated tumor cells (DTCs, disseminated tumor cells), endometrial cells and circulating trophoblast cells (CFTC, circulating fetal trophoblast cells), allowing subsequent detection, quantification, characterization and especially culturing.

### BACKGROUND OF THE INVENTION

Metastatic lesions are the most common cause of death in patients with carcinomas (Birchmeier, 1996). During metastasizing tumor cells detach from the primary tumor and enter the blood stream directly or through the lymphatic system, migrating into secondary organs and developing a metastatic deposit. Detection of CTCs in patients with metastatic carcinoma is associated with a worse prognosis (Zharo 2011 , Zhang 2012 , Wang 2011). Despite their great clinical significance, a molecular characterization of CTCs is still not performed. This can be attributed to extremely low frequency of CTCs compared with the number of blood cells. CTCs are generated by the process of epithelial-mesenchymal transition (EMT), which is characterized by reduced expression of epithelial markers and increased expression of mesenchymal markers. These changes promote increased motility, invasion and resistance to therapy (Thiery 2002 Shook 2003 Christofori 2006 Jechlinger 2003). CTCs/DTCs represent a population of tumor cells disseminated to distant organs bearing the potential for micro- and macro-metastatic foci creation. Some of the tumor cells from primary tumors and/or CTCs and DTCs have properties of stem cells (CSCs, cancer stem cells). The population of cancer stem cells has the ability to self-regeneration and is resistant to treatment, meaning a worse prognosis.

CTCs/CSCs have enormous potential in the diagnosis, determining prognosis, monitoring disease therapeutic effect and risk (Mani 2008).

Early dissemination of the tumor to lymphatic nodes and blood is realized by circulating tumor cells (CTCs), and disseminated cancer cells (DTCs). The CTCs have been described as also occuring in patients due to resection of the primary tumor simple by handling with the primary tumor.

Detection of CTCs is unquestionably a prognostic factor in cancer. CTCs detection can potentially be used to diagnose or as an alternative to invasive biopsies for early detection of metastatic spread of the tumor, and for setting and monitoring the effectiveness of therapy in individual patients. The quantity and characteristics of CTCs may be a prognostic factor for survival or predictive factor of a therapy response. Long-term characterization of CTCs may provide an opportunity to better assess the dynamic physiological response. Also, changes in the number of CTCs in comparison to the previously monitored changes found by imaging suggest that CTCs analysis may be more important for the assessment of survival than imaging methods.

In general, there is a valid evidence of adverse prognostic value of CTCs detected in metastatic colorectal cancer, prostate cancer, ovarian cancer, breast cancer. There are also studies on the significance of CTCs in the formation of venous thromboembolism. In patients with metastatic breast cancer and with detected CTCs (n ≥ 1) in peripheral blood up to four times higher incidence of thrombosis was observed when compared with patients without CTCs (Mego 2009). CTCs are possibly involved in the activation of coagulation through the expression and release of tissue factors (Davila 2008). CTCs are present in the blood in a very small amount of approximately one cell to ten billion blood cells (Pantel 2001, Ziegelschmidt 2005).

Currently available technologies use different methods for detection of CTCs: a density gradient separation, an immuno-magnetic separation, a gradient immuno-magnetic separation. These methods are specific to cells and more gentle than pressure filtration methods, but upon binding the antibody interacts with receptors and antigens on the cell membrane, and thus authentic and viable CTCs/DTCs are difficult to select.

The presence of sporadic cells may also be detected indirectly by RT-PCR, but this approach is often limited by a low expression of tumor-specific markers and the non-specificity of the antigens typical for CTCs and DTCs (Andreopoulou 2012). Additionally, a high degree of discrepancy in expression of surface antigens between primary tumors and CTCs/DTCs has been proved, suggesting great unreliability in the use of these markers for the CTCs/DTCs separation.

Given the size of the separated sporadic CTC cells in most epithelial tumors is much larger than the blood cell size, one of the efficient ways of the CTCs separation is based on the filtration process through a filter membrane, wherein the separated cells remain on the membrane and other cell pass through the membrane (Paterlini-Brechot et al. 2007, Vona 2000). Using a size-based separation method of CTCs/DTCs allows the detection and separation of CTCs/DTCs without dependence on surface antigens and receptors, whose expression varies during a cancer disease.

Recently used filtration methods include a pressure- or vacuum-accelerated filtration process (Mikulova, 2011); the filtration gradient is thus created by changing the pressure. The common disadvantage of filtration methods is that there is an accumulation of a precipitate, and a precipitation on the filter membrane, causing the filter clogging, which leads to capturing the blood cells in the precipitate. Consequently, the precipitate and the blood cells considerably deteriorate the evaluability of the presence of the sporadic cells on the filter under the microscope. The captured blood cells may be partially removed by washing, however the precipitate remains on the filter. For this reason, additional anticoagulant agents are to be added into the filtering apparatus (not only into the tube during blood sampling). A fundamental disadvantage, as with most other methods, is damaging the viability of the cells, so the cells do not usually further divide and their subsequent cultivation impossible.

The documents WO 2006/116327 A1 and US 2012/0178097 A1 disclose membrane filters for separation of viable circulating tumor cells (CTCs), which are biocompatible filters made of parylene or polycarbonate of pore size 5 to 40 micrometers.

The documents US 2010/324449 A1 and US 5114350 A disclose filters contacting an absorbent material. All currently used methods have also other disadvantages, e.g. require analysis of large volumes of blood, a large proportion of laboratory work, time-consuming evaluation (sample processing takes several hours), or costly equipment and reagents used, and/or the methods lack reliability, sensitivity, efficiency, specificity and standardization necessary for routine CTCs and DTCs detection methods.

A method would therefore be desirable for separating cells, especially sporadic cells from body fluids, said method being reproducible, showing good evaluation of the sporadic cells presence on the filter under a microscope, which is not processing time-, equipment- and operating personnel-demanding, which is inexpensive even at low numbers of tested samples, and which does not require expensive reagents with limited expiration. In the process of separation there should not occur any interaction with receptors and antigens. The process should be gentle to the cells in order to eanble the selection of viable cells and authentic CTCs/DTCs and to use said cells in subsequent experiments in vitro and in vivo, possibly.

Furthermore an easily manufactured device would be desirable, for carrying out the isolation process reproducibly, being affordable to the most of the laboratories, suitable for sterile handling of potentially pathological material. Said device should provide a possibility for subsequent detection and/or quantification and/or culturing separated cells on a filter membrane, and can be available as a disposable or reusable variant.

### SUMMARY OF THE INVENTION

The shortcomings of the prior methods were removed and the set requirements were fulfilled by a method of separation of sporadic cells from body fluids of human and animal organisms according to the present invention utilizing a filter membrane positioned in an intimate contact with an absorbent material.

It was found out that in contrast to the pressure/vacuum filtration, during the filtration utilizing the capillary force (capillarity) clotting of proteins from body fluids such as blood or blood plasma occurs to a minimum extent, so there is no need for an additional anticoagulant to the anticoagulant (EDTA) added to blood sampling tube for a normal blood collection. If there a clot in the body fluid is already present, dilution by a small amounts of a buffer such as PBS is sufficient to prevent clotting. Consequently the filter membrane is not being clogged (particularly at the beginning of the filtration, as normally happens in the pressure/vacuum filtration), and the separation is smoother, faster and complete.

A lower amount of the precipitate brings about a lower amount of blood cells such as red blood cells retained in said precipitate, wherein in principal both the precipitate as such and the blood cells destroy detection of the presence of the sporadic cells on the filter. Obviously the correct evaluation of the presence of sporadic cells is crucial; the filtration time can quite easily be adjusted by the filtration area used, i.e. the area of the filter membrane and of the absorbent material

Furthermore, it was found that the sporadic cells captured on the membrane remain mechanically and chemically unchanged and therefore viable, which enables their subsequent effective cultivation, which is not practicable by other methods, from the perspective of current results.

Furthermore, it was found that for dilution of the body fluid or for rinsing of sporadic cells on the filter respectively, the culture medium such as RPMI can directly be used in order to increase the success rate of the separation (and also of the cultivation, if the aim in also to further cultivate the sporadic cells), while the separation velocity on the filter membrane as well as the cell viability are retained.

The filtered body fluid free from the sporadic cells passes through the holes in the filter membrane, wherein the elements larger than the holes in the filter membrane are captured (typical size of blood cells is under 6 µm and that of sporadic cells above 10 µm).

The separated sporadic cells are lodged due to their size on the filter membrane, which membrane can then be separated from the absorbent material, having the membrane still fixed in the holder. In another embodiment, the membrane may be subsequently completely removed from the holder.

The flow of body fluids, free from sporadic cells, through the membrane is continuously accelerated by virtue of capillary force sucking the liquid into the absorbent material. There is no overpressure before the filter and no underpressure (vacuum) behind the filter applied to the filtered body fluid during the filtration. The fluid is only sucked by capillary force, without the need for any regulation, at appropriate speed through the filter to the absorbent material. (In this context, the hydrostatic pressure exerted on the filter by the height of the body fluid column is disregarded). The amount of the capillary force causes continuous filtering of the fluid, without any clogging before or on the filter membrane, as is frequently observed, for example, in methods using vacuum behind the filter or an overpressure before the filter.

The advantage of the solution according to the invention is high efficiency of capturing of even low concentrated sporadic cells and accelerating of the separation process, thus also the detection speed and increasing of the cultivation efficiency. When using the method according to the invention, no interaction of sporadic cell with receptors and antigens is observed, and it is therefore possible to select authentic and viable CTCs/DTCs, which can then be used in experiments *in vitro* and then *in vivo.* The method allows keeping the filtered cells in very good vital condition for further analysis. No special laboratory equipment is needed. The method allows processing of relatively large volumes of blood or other body fluids (e.g. 50 ml).

Accordingly, the present invention provides a method for separation of sporadic cells present in the patient's body fluid by their capturing on a filter membrane having openings smaller than the diameter of the cells, wherein from the sporadic cells present in said body fluid present on the first side of the filter membrane, the body fluid free of sporadic cells is drawn through the filter membrane by virtue of capillary force into an absorbent material positioned in an intimate contact with the second side of said filter membrane.

Sporadic cells present in the body fluid are for example circulating tumor cells, disseminated tumor cells, endometrial cells and circulating fetal trophoblastic cells.

The body fluid is any fluid obtained directly (by collection) or indirectly (with subsequent treatments of any type) from the human or animal body. It is preferably selected from the group of central or peripheral blood, bone marrow, ascites fluid, pleural effusion, peritoneal lavage fluid and amniotic fluid.

The test object can preferably be a patient with primary cancer or metastatic disease.

The test sample can also be successfully collected from an animal that has been approved as an experimental metastatic model.

In another embodiment, the peripheral blood is anticoagulated peripheral blood, such as blood collected into a regular blood collection tube comprising anticoagulant such as EDTA.

If the body fluid comprises precipitate prior to separation, the filtered body fluid is preferably diluted with an appropriate buffer, such as PBS (physiological salt solution with phosphate buffer) and/or TrypLE™ in order to dissolve the precipitate and to make the filtration/separation easier.

The invention, however, does not exclude the use of any known methods for preventing the blood clot formation during the blood collection. An example is a gradual loading and optionally enzymatic dissolution (e.g. by using TrypLE™, Invitrogen), however preferably the dissolution is not used.

To improve filtration in case of presence of blood precipitates, the peripheral blood may be further diluted by PBS solution or by the cultivation medium. In order to remove blood precipitates and other coarse impurities, blood or other body fluids may also be filtered through a sieve with the pore size of e.g. 0.1 mm, which is then washed several times with PBS. Thereafter the residual blood volume is loaded. Finally, the blood collecting tube is rinsed with PBS, the volume of which is filtered through the sieve.

The separated sporadic cells may then be rinsed on the filter for better microscope evaluation, e.g. by the buffer such as PBS or by a medium such as RPMI. Compared with vacuum filtration, the method according to the invention provides substantial benefits in all aspects.

The separated cells may then be further analyzed, e.g. microscopically, preferably after immunohistochemical or other staining, quantified (e.g., microscopically, manually or automatically) or cultured or otherwise processed. In other embodiments, a detection and/or quantification and/or cultivation of the separated cells is carried out following the separation, directly on the filter membrane or in a culture vessel.

The filters with sporadic cells may be stored for longer period frozen, dried or fixed by conventional methods in use to be analyzed later.

As already stated, especially in the case where the subsequent culturing of sporadic cells is planned, the body fluid may be diluted directly by the culture medium used in the subsequent cultivation of separated cells prior to the separation, such as RPMI medium, thereby further increasing the success rate of filtration and the subsequent cultivation.

The subsequent cultivation of sporadic cells can be carried out in conventional manner in an incubator at 37 °C and 5% CO₂ in a conventional culture dish or a plastic or glass bottle upon washing the cells off the filter. For this purpose, for example, the filter is rinsed with 2 x 1 ml of the RPMI medium, and the medium with cells is transferred to a 24-well plate, where the cells may be cultured directly on the surface of the plastic plate or on the inserted microscope coverslip. The coverslip culture is preferred if immunohistochemistry and immunofluorescence analysis of the cells is required.

Cultivation is preferably carried out by inserting the filter containing captured cells to the culture medium in a culture dish, or by pouring the culture medium to the filter with the captured cells, inserted in the culture dish.

In another preferred embodiment, the cultivation is carried out by transferring the filter membrane mounted in the holder described below, immediately after the separation, for example, into a 6-well culture plate with a growth medium added into each well. The cells can then be cultivated as a standard tissue culture.

In one embodiment of the invention, a body fluid is the body fluid of patients with melanoma, breast cancer, cancers of the gastrointestinal tract such as gastric cancer, colon cancer, pancreas cancer and liver cancer; urogenital tumors, and soft tissue tumors such as head and neck tumors, and other solid tumors.

The method and apparatus of the present invention may be used, for example, for separation of circulating tumor cells (CTCs) from peripheral or central blood, tumor cells of the gastrointestinal tract (esophagus, stomach, large and small intestines, liver, pancreas, gallbladder and biliary tract), tumors of the urogenital system (ovary, endometrium, prostate, bladder, testis), tumors of the respiratory system (lung, mediastinum, upper respiratory tract), tumors of the neuroendocrine system, bone tumors, head and neck tumors, breast tumors, metastatic tumors without the primary localization, and other less frequent solid tumors.

The method and apparatus according to the present invention can also be used for selected hematopoietic diseases, neuroendocrine tumors, disseminated tumor cells from ascites, from pleural effusion, from sputum, from lavages (bronchoalveolar lavages, lavages of peritoneum and chest cavity, lavages of retroperitoneum, etc.), for circulating and disseminated endometrial cells (from blood and from lavages).

An important utilization also applies to the separation of circulating fetal trophoblast cells (CFTCs) from the blood, or amniocentic fluid, because CFTCs are present in the peripheral maternal blood from the 5th gestation week and they do not remain in blood circulation after the end of pregnancy (Bianchi et al., 1996). CFTC are therefore very attractive source of DNA/RNA for examination by a non-invasive prenatal diagnosis (PND-NI). By comparing the tests based on CFTC and choriocentesis both 100 % diagnostic sensitivity and specificity has been confirmed for testing on the basis of CFTC. The basic feature by which CFTC cells differ from blood cells is their size (CFTC cells reach a standard size 10 µm and more). Current reports indicate that a pregnant woman has a 99% probability that each cell of the size over 15 µm size, the cell is of embryonic origin, thus CFTC (Mouawia, 2012). Due to the new methodology, it is now possible to reproducibly separate undamaged CFTC and use them for further analysis.

The detection of CTCs and DTCs can be carried out in animal tumor models, and CTCs/ DTCs can be detected in individuals with more than one type of tumor.

After separation, the cells may be processed by both immunohistochemistry methods and single-cell methods for gene expression.

The invention further provides an apparatus for performing the separation of a mixture of sporadic cells present in the body fluid of a patient by the methods described above, which apparatus comprises a filter membrane positioned in an intimate contact with an absorbent material.

The geometry of the membrane generally involves the size, shape and density of the membrane pores. The effectiveness of the membrane filter can thus be optimized by adjusting the size, shape and density of the membrane pores. Such a construction of the membrane is selected, which prefers capturing of the tumor and sporadic cells over the blood cells. The filter membrane is preferably such a membrane that captures sporadic cells of the dimensions of above 10 µm and transmits blood elements of the size up to 6 µm, i.e. the membrane having the pore size of 7-10 µm, e.g. 8 µm or similar value e.g. within the range ± 1 µm declared by the manufacturer. The filter membrane is preferably made of a biocompatible material such as polycarbonate, which offers the advantages of flexibility and biocompatibility.

Minimizing the effects of clotting and increasing the filtration velocity can be further achieved by using a larger filter with the largest available filtering surface. Polycarbonate membranes with a pore size of 8 µm with membrane diameter of 25 mm can be used, for example.

The term "an intimate contact" refers to such a close contact of the filter membrane and the absorbent material that the capillary action through the membrane into the absorbent material is not affected, namely an access of air into the interface between the membrane and the absorbent material, which is in the direct contact with the filtered liquid only through the pores in the membrane, is prevented during the operation. This can be achieved e.g. by pushing the edges of the membrane, or the membrane clamped into a suitable apparatus, to the surface of the absorbent material, or by pushing the edges of the membrane mounted in a suitable holder under slight pressure into the flexible surface of the absorbent material.

The absorbent material has sufficient absorption capacity and is able to smoothly absorb all blood elements and other elements that are present in the body fluid of variable viscosity, and the absorbent mainly consists of pulp, fine paper, textile fibers, or combinations thereof in particular.

The volume of the filtered body fluid is limited by the suction capacity of the absorbent material and by its volume. Size and/or volume of the apparatus and characteristics of the absorbent material can therefore be adapted to the intended use. Advantageously, using a single apparatus a volume of 50 ml and more can be filtered. The method and the apparatus work even from a minimum volume of blood, such as less than 1 ml, however, with increasing volume of filtered body fluid a probability of capturing the sporadic cells increases.

The apparatus according to the invention preferably further comprises a top- and bottom-open hollow container for receiving a mixture of sporadic cells present in the body fluid, wherein the lower periphery of the container is in tight contact with at least a part of the upper first side of the membrane, and wherein at least a part of the lower second side of the membrane is in an intimate contact with the absorbent material so that the membrane separates the inner space of the container from said absorbent material. The upper edge of the container may preferably be broadened to obtain a funnel and/or said edge can be provided with a sieve to remove clots already present in the blood, etc.

From the above reason, to achieve maximum filtration area at least part of the first side of the membrane and at least part of the second side of the membrane are placed against each other over the largest area that maximizes the contact area between the absorbent material and sporadic cells present in the body fluid, separated by a membrane.

In another embodiment, the absorbent material is arranged in a collecting base container provided with an upper lid, into which lid from above a circumferential membrane holder releasably engages, and wherein said holder, by means of a pressure ring, tightly releasably holds the membrane along its circumference, and wherein to the reservoir from above the holder is tightly attachable along the circumference of said holder. The apparatus is preferably designed for a single use, but neither an embodiment of the apparatus for reuse all elements except the membrane and absorbent material is excluded.

### DESCRIPTION OF THE DRAWINGS

FIG. 1 illustrates one embodiment of a filter apparatus in two views
FIG. 2 shows an embodiment of a filter apparatus in an expanded wiew (the absorbent material is not shown)
FIG. 3 illustrates one embodiment of fastening the membrane in the holder
FIG. 4 shows an *in vitro* cell culture of CTCs separated from peripheral blood of patient with prostate cancer, CTCs grown on a filter membrane
FIG. 5 shows the *in vitro* culture of CTCs on the filter membrane, May-Grünwald staining method has been used for haematological analysis

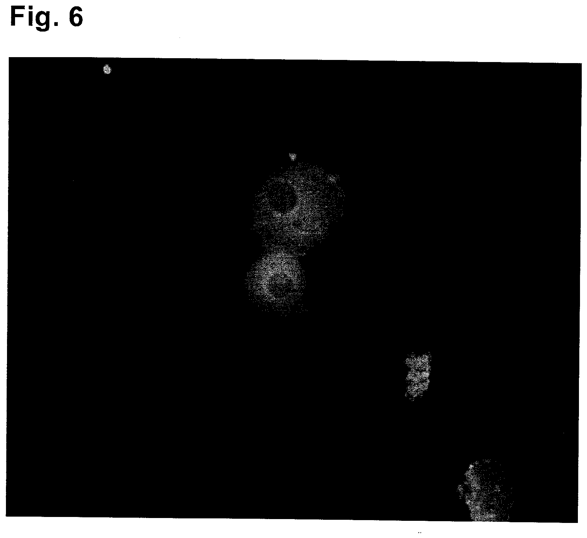

FIG. 6 illustrates the processing of the separated cells by imunohistochemistry, (CTCs isolated from blood of a patient with metastatic prostate cancer stained by antibody against the pan-cytokeratin)

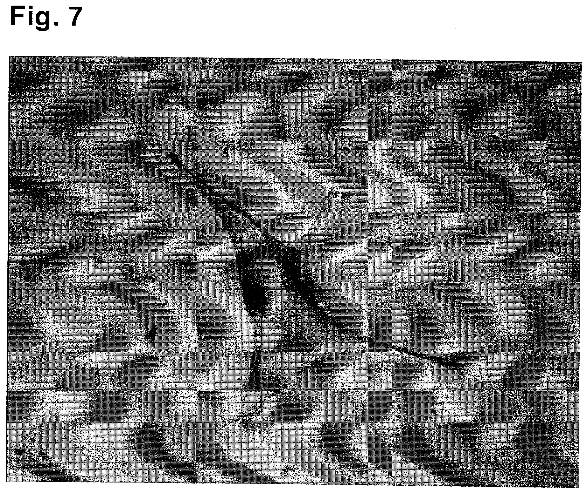

FIG. 7 shows a pair of separated CTCs, which in the course of cultivation penetrated the filter on the bottom of the culture wells (May-Grünwald staining method has been used for analysis).

### EXAMPLES

### Abbreviations:

- CTCs: circulating tumor cell
- DTCs: disseminated tumor cell
- CFTC: circulating fetal trophoblast cells
- NI-PND: non-invasive prenatal diagnosis
- PBS: phosphate buffer solution with 0.15 M NaCl, essentially buffered saline
- FBS: fetal bovine serum
- EDTA: Chelaton 2, ethylenediaminetetraacetic acid

### Example 1

### Determination of circulating tumor cells in prostate cancer from the peripheral blood of patients undergoing surgical removal of the primary tumor

5-10 ml of peripheral blood is collected during surgery into a collecting tube with EDTA (e.g. Vacuette K3E (REF 456 036), S-monovette K3E (REF 02,1066,001). Blood is processed immediately or stored at 4 °C for a maximum of 24 hours.

Peripheral blood is gradually applied by the pipette to the container 3 of the apparatus for performing the filtration. The blood present on the first side of the membrane 1 is filtered through the filter membrane 1 of the diameter of 25 mm consisting of a polycarbonate membrane (PCTE) having a pore size of 8 microns (GE, Polycarbonate, 8.0 Micron, 25mm), wherein the filter membrane 1 is in an intimate contact with the absorbent material 2, the pulp (Pur-Zellin Hartmann). The filtration process takes ca 5 min, depending on the volume of the filtered blood and the blood viscosity.

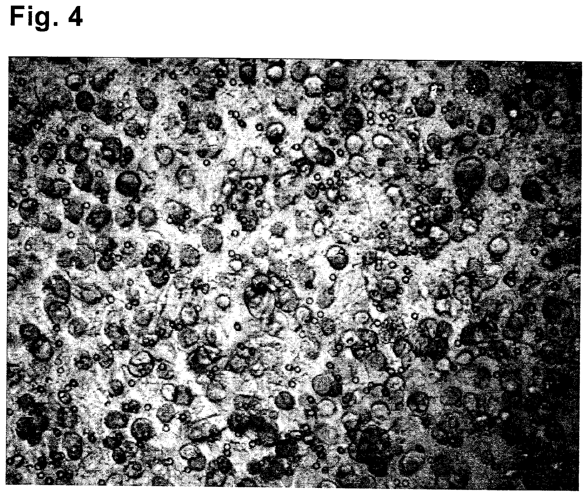

Further, it is possible to proceed in several ways:
1. After filtration, the filter membrane 1, fixed in a holder 6 and comprising isolated CTCs, is transferred into one well of a 6-well culture plate into which the RPMI growth medium (4 ml), RPMI (Sigma R8758), enriched with FBS (F2442, Sigma) and antibiotics (Penicillin-Streptomycin (P4458, Sigma), Amphotericin (A2942), Sigma) are added. The captured cells are then cultured as normal tissue culture for at least 14 days at 37 °C, 5% CO₂, with a medium exchange every 3 days.
   The grown cell culture was stained according to the standard protocol of May-Grünwald immunohistochemical staining and a photograph from inverted light microscope is shown in Figure 4.

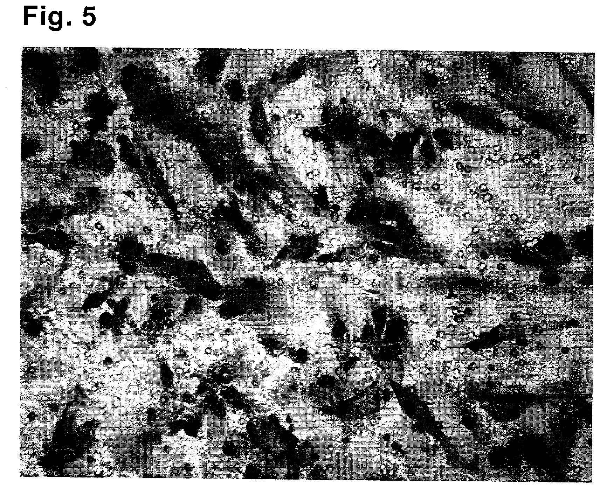

   Figure 5 shows a similar arrangement of a variation of the immunofluorescence staining of CTCs in prostate cancer (pan-cytokeratin-FITC, Sigma), covered with Prolong Gold™ with DAPI).
2. After filtration, the filter membrane 1 comprising isolated CTCs is rinsed with 1 ml medium, and said medium with the cells washed out from the filter is then transferred into a 24-well plate where it is overlaid on a microscope slide placed on the well bottom (Assistent, N.1001, 12 mm diameter). The cells then grow directly on the microscope slide, which further facilitates handling during the immunohistochemical staining and the subsequent immunohistochemical analysis, at least 14 days at 37 °C, in a 5% CO₂ atmosphere, with medium change every 3 days.
3. After filtration, the filter membrane 1 comprising isolated CTCs is rinsed with 1 ml of the medium, the medium with the cells from the membrane is then transferred by pipette into culture chambers (Nunc® Lab-Tek® ChamberSlide™ (4 chambers) for confocal microscopy (the system for real-time video capturing Time-lapse imaging, Leica) and then cultured as normal tissue culture at least 14 days at 37 °C, 5% CO₂ atmosphere, with medium change every 3 days.

In all above mentioned examples, the cultivation of both sporadic cells on the membrane and sporadic cells rinsed off the membrane was carried out without any problems. Likewise, the cultivation of cells from other tumor types isolated by the method of the present invention was carried out without any problems. By contrast, successful culturing of sporadic cells, separated from body fluids of patients by methods known in the art both on a membrane and without the membrane, has not yet been evidenced.
4. After filtration and rinsing of sporadic cells captured on the filter by 2 ml RPMI, the filter membrane 1 mounted in a holder containing isolated CTCs is stained directly by standard May-Grünwald staining protocol and the presence of sporadic cells is evaluated under the microscope.

### Example 2

### Determination of disseminated tumor cells in the metastatic ovarian cancer of the pleural effusion

Disseminated tumor cells from the pleural effusion removed from a patient with metastatic ovarian cancer were separated as follows: the effusion was applied by a pipette into the container 3 of the filtration apparatus. The effusion on the first side of the membrane 1 is filtered through the filter membrane 1 of 25 mm diameter made of polycarbonate membrane (PCTE) having the pore size of 8 µm as in Example 1, wherein the absorbent material 2, pulp (Pur-Zellin,Hartmann) is immediately adjacent to the filter membrane 1. The cells were fixed to the filter and analysed as hematological blood stains, stained by standard May-Grünwald staining protocol and evaluated under the microscope.

### Example 3

### Apparatus for performing filtration

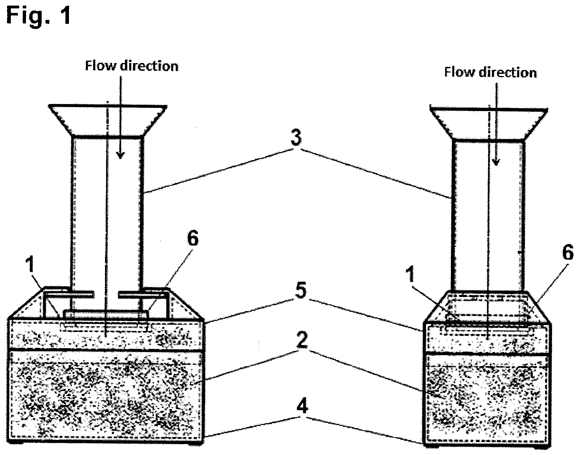

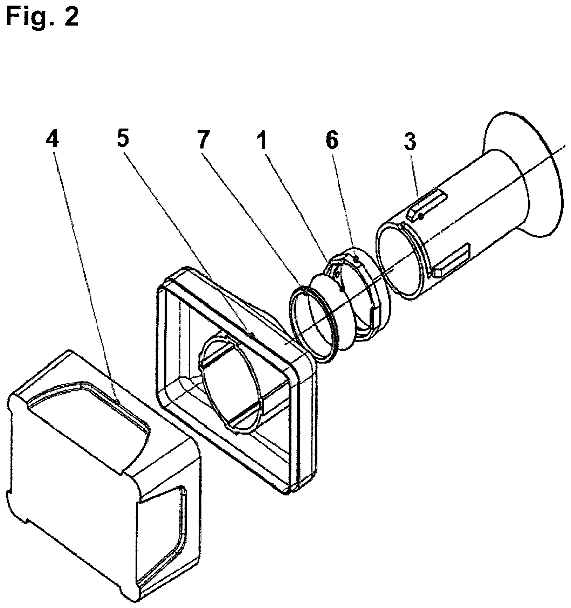

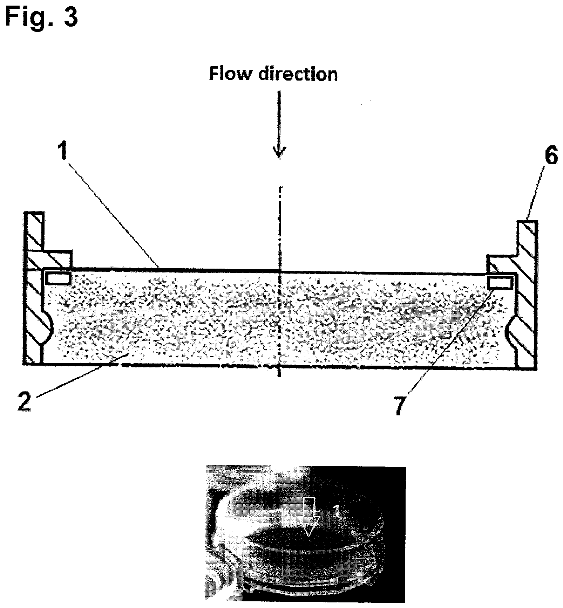

One embodiment of the apparatus for performing the filtration shown in Figures 1-3 comprises a polycarbonate filter membrane 1, with a pore size of 8 µm, which the absorbent material 2 consisting of pulp is in an intimate contact with, from the bottom side of said membrane. The apparatus further includes a top- and bottom-open hollow container 3 for receiving a mixture of sporadic cells present in the body fluid. For easier pouring of liquids the upper edge of the container 3 is broadened so as to form a funnel. The lower periphery of the container 3 is in tight contact with the upper first side of the membrane 1, and the lower second side of the membrane 1 is in an intimate contact with the absorbent material 2, so that the membrane 1 separates the inner space of the container 3 from said absorbent material 2.

The absorbent material 2 is arranged in a collecting base container 4 provided with an upper lid 5, into which lid a circumferential membrane holder 6 releasably engages from above. The holder 6 is vertically movable in relation to the lid 5 and therefore also to the absorbent material 2, which allows the intimate contact of the filter membrane 1 to the absorbent material 2, or pushing of the pressure ring 7 into the absorbent material 2 respectively for perfect contact of the membrane 1 and absorbent material 2. The holder 6 holds the membrane 1 along its circumference by the pressure ring 7 tightly releasably using a thread, so that the set of the holder 6, filter membrane 1, and pressure ring 7 can be removed from the apparatus as a whole, and the captured cells can be stained or cultured directly on the membrane 1. The container 3 is releasably attached by a bayonet connection to the holder 6 along its circumference.

### Example 4

### Comparison of two kinds of filter technology in relation to the presence of a precipitate

The aim of the experiment was to compare the filtration of blood cells containing sporadic cells accelerated by vacuum with the filtration using capillarity forces according to the invention in relation to an evaluation of the presence of sporadic cells collected on the filter membrane, which mainly depends on the presence of a precipitate and residual blood cell elements on the filter membrane.

Both processes use a membrane with a declared pore size of 7-8 µm and an absorbent material as in Example 1. In order to achieve approximately equal filtration times different sized filters were used - the filter with diameter of 7 mm was used for vacuum filtration and the filter of diameter of 25 mm for a filtration using capillary forces.

The same volume of 2 ml of blood obtained as in Example 1 from a patient with prostate cancer was filtered through the two membrane systems at room temperature, with the filtration times 87 s for the filtration using the vacuum system and 97 s for the filtration using capillary forces according to the invention.

A frequent occurrence of blood precipitate on filters has been observed in the case of the vacuum filtration, which made impossible assessing the presence of sporadic cells on the filter during microscopy. When using the filtration method by capillary forces according to the invention no precipitate was present on the filter.

### Example 5

### Comparison of two kinds of filter technology from a viewpoint of rinsing

The same configuration was used as in Example 4 except that the blood sample was taken from a patient with breast cancer. Since we frequently encountered clot formation during vacuum filtration in this type of samples, leading to difficult evaluation mainly due to red blood cells trapped in the precipitate, the filters were washed with additional 2 ml of growth medium (RPMI) after filtration of blood.

As expected, precipitate remained on the filter after vacuum filtration, but the rinsing helped to remove the residue of red blood cells, and thus the overall evaluation of presence of sporadic cells on the filter after rinsing with RPMI is better than in the case without rinsing. Total filtration and rinsing time for the filter with an RPMI medium was 4 min 37 s.

After using capillary forces for filtration the filter showed better evaluation even without rinsing than the filter after the use of vacuum. The evaluation of the presence of sporadic cells on the filter after rinsing with RPMI improved further and was better than the evaluation of the rinsed filter using vacuum. The total filtration and rinsing time for the filter with RPMI medium was only 39 s in this case.

Accordingly, the advantage of the use of capillary forces for filter separation of sporadic cells both in view of the occurrence of clots, and also in view of the evaluation of presence of sporadic cells, was confirmed. Moreover, a reduction in the filtration time was observed in the step of rinsing with RPMI medium.

## Claims

1. A use of a method for separation of sporadic cells, present in the body fluid of a patient, by capturing them on a filter membrane having openings smaller than the diameter of the cells, in which from the sporadic cells present in said body fluid present on the first side of the filter membrane, the body fluid free of sporadic cells is drawn through the filter membrane by virtue of capillary force into an absorbent material positioned in an intimate contact with the second side of said filter membrane, to obtain said sporadic cells captured on the membrane remaining mechanically and chemically unchanged and therefore viable, which enables their subsequent effective cultivation.

2. The use according to claim 1, **characterized in that** the sporadic cells present in the body fluid are circulating tumor cells, disseminated tumor cells, endometrial cells and circulating fetal trophoblast cells.

3. The use according to claim 1 or 2, **characterized in that** the body fluid is selected from peripheral or central blood, bone marrow, ascites fluid, pleural effusion, peritoneal lavage fluid, bronchoalveolar lavage and amniotic fluid.

4. The use according to any of the preceding claims, **characterized in that** said body fluid is a body fluid of patients with melanoma, breast cancer, tumors of the gastrointestinal tract such as gastric carcinoma, colon carcinoma, pancreas carcinoma and liver carcinoma; urogenital tumors and soft tissue tumors such as head and neck tumors and other solid tumors.

5. The use according to claim 3, **characterized in that** the peripheral blood is collected as anticoagulated peripheral blood.

6. The use according to any of the preceding claims, **characterized in that** prior to separation the precipitate containing body fluid is diluted with a buffer, in particular with PBS, in order to dissolve the precipitate.

7. The use according to any of the preceding claims, **characterized in that** subsequent to the separation, detection and/or quantification and/or culturing the cells separated on the filter membrane, or the cells flushed out of the filter membrane in a culture vessel is carried out.

8. The use according to claim 7, **characterized in that** before separation the body fluid is diluted with a culture medium, RPMI in particular, to increase the success of subsequent cultivation.

9. An apparatus for separation of sporadic cells, present in the body fluid of a patient, by capturing them on a filter membrane having openings smaller than the diameter of the cells, in which from the sporadic cells present in said body fluid present on the first side of the filter membrane, the body fluid free of sporadic cells is drawn through the filter membrane by virtue of capillary force into an absorbent material positioned in an intimate contact with the second side of said filter membrane, to obtain said sporadic cells captured on the membrane remaining mechanically and chemically unchanged and therefore viable, which enables their subsequent effective cultivation., **characterized in that** said apparatus comprises a top- and bottom-open hollow container (3) for receiving a mixture of sporadic cells present in the body fluid, wherein the lower periphery of the container (3) is in tight contact with at least a part of the upper first side of the membrane (1), said membrane having the pore size of 7-10 µm and being made of a biocompatible material, and wherein at least a part of the lower second side of the membrane (1) is in an intimate contact with the absorbent material (2) so that the membrane (1) separates the inner space of the container (3) from said absorbent material (2).

10. The apparatus according to claim 9, **characterized in that** the absorbent material (2) is arranged in a collecting base container (4) provided with an upper lid (5), into which lid a circumferential membrane holder (6) releasably engages from above, and wherein said holder (6), by means of a pressure ring (7), tightly releasably holds the membrane (1) along its circumference, and wherein to the reservoir (3) from above the holder (6) is tightly attachable along the circumference of said holder (6).

11. The apparatus according to claim 9 or 10 **characterized in that** the filter membrane (1) has a pore size of 8 µm

12. The apparatus according to any of claims 9 to 11, **characterized in that** the filter membrane (1) is made of polycarbonate.

13. The apparatus according to any of claims 9 to 12, **characterized in that** the absorbent material (2) consists of cellulose, fine paper, textile fibers, or a combination thereof.

14. The use according to claim 7, **characterized in that** for carrying out the detection and/or quantification and/or cultivation, the separated cells are transferred on the filter membrane (1) fixed in a holder (6) of claim 10.

## Patentansprüche

1. Verwendung eines Verfahrens zur Separation von in der Körperflüssigkeit eines Patienten vorkommenden sporadischen Zellen durch Einfangen von diesen Zellen auf einer Filtermembran, die Öffnungen kleiner als der Zelldurchmesser aufweist, wobei von den in der auf der ersten Seite der Filtermembran vorkommenden Körperflüssigkeit vorkommenden Zellen die die sporadische Zellen nicht enthaltende Körperflüssigkeit durch die Filtermembran unter Wirkung von Kapillarkraft in ein Absorbens, das im engen Kontakt mit der anderen Seite der genannten Filtermembran angebracht ist, angesaugt wird, um die genannten auf der Membran eingefangenen sporadischen Zellen zu erhalten, die mechanisch und chemisch unverändert und also lebensfähig werden, was ihre anschließende effektive Kultivierung ermöglicht.

2. Verwendung nach Anspruch 1, **dadurch gekennzeichnet, dass** die in der Körperflüssigkeit vorkommenden sporadischen Zellen zirkulierende Tumorzellen, disseminierte Tumorzellen, Endometriumzellen und zirkulierende embryonale Trophoblastzellen sind.

3. Verwendung nach Anspruch 1 oder 2, **dadurch gekennzeichnet, dass** die Körperflüssigkeit aus peripherem oder zentralem Blut, Knochenmark, Aszitesflüssigkeit, Pleuraeguss, Bauchspülungsflüssigkeit, bronchoalveoläre Spülung und Fruchtwasser ausgewählt wird.

4. Verwendung nach einem der vorangegangenen Ansprüche, **dadurch gekennzeichnet, dass** die Körperflüssigkeit eine Körperflüssigkeit von Patienten mit Melanom, Brustkrebs, Tumoren vom Magen-Darm-Trakt wie Magenkarzinom, Dickdarmkarzinom, Pankreaskarzinom und Leberkarzinom; urogenitalen Tumoren und Weichgewebetumoren wie Kopf- und Halstumoren und anderen festen Tumoren ist.

5. Verwendung nach Anspruch 3, **dadurch gekennzeichnet, dass** peripheres Blut als antikoaguliertes peripheres Blut abgenommen wird.

6. Verwendung nach einem der vorangegangenen Ansprüche, **dadurch gekennzeichnet, dass** vor der Separation das die Körperflüssigkeit enthaltende Präzipitat mit einem Puffer verdünnt wird, besonders mit PBS, um das Präzipitat aufzulösen.

7. Verwendung nach einem der vorangegangenen Ansprüche, **dadurch gekennzeichnet, dass** nach der Separation Detektion und/oder Quantifizierung und/oder Kultivierung der auf der Filtermembran separierten oder der von der Filtermembran in ein Kulturgefiäß ausgespülten Zellen durchgeführt wird.

8. Verwendung nach Anspruch 7, **dadurch gekennzeichnet dass** vor der Separation die Körperflüssigkeit mit einem Kulturmedium, besonders RPMI verdünnt wird, um den Erfolg der nachfolgenden Kultivierung zu erhöhen.

9. Eine Einrichtung zur Separation von in der Körperflüssigkeit eines Patienten vorkommenden sporadischen Zellen durch Einfangen von diesen Zellen auf einer Filtermembran, die Öffnungen kleiner als der Zelldurchmesser aufweist, wobei von den in der auf der ersten Seite der Filtermembran vorkommenden Körperflüssigkeit vorkommenden Zellen die die sporadische Zellen nicht enthaltende Körperflüssigkeit durch die Filtermembran unter Wirkung von Kapillarkraft in ein Absorbens, das im engen Kontakt mit der anderen Seite der genannten Filtermembran angebracht ist, angesaugt wird, um die genannten auf der Membran eingefangenen sporadischen Zellen zu erhalten, die mechanisch und chemisch unverändert und also lebensfähig werden, was ihre anschließende effektive Kultivierung ermöglicht, **dadurch gekennzeichnet, dass** die Einrichtung einen oben und unten geöffneten Behälter (3) zum Aufnehmen eines Gemisches der in der Körperflüssigkeit vorkommenden sporadischen Zellen aufweist, wobei der untere Rand des Behälters (3) im engen Kontakt mit mindestens einem Teil der oberen ersten Seite der Membran (1) ist, wobei die genannte Membran eine Porengröße von 7-10 pm aufweist und wobei mindestens ein Teil der unteren zweiten Seite der Membran (1) im engen Kontakt mit dem Absorbens (2) ist, sodass die Membran (1) den Innenraum des Behälters (3) vom genannten Absorbens (2) trennt.

10. Die Einrichtung nach Einspruch 9, **dadurch gekennzeichnet, dass** das Absorbens (2) in einem Grundsammelbehälter (4) angeordnet ist, der einen oberen Deckel (5) aufweist, in den ein umlaufender Membranhalter (6) lösbar von oben eingreift und wobei der genannte Halter (6) mittels eines Druckringes (7) fest und lösbar die Membran (1) entlang ihres Umfangs hält und wobei der Halter (6) fest aufsetzbar von oben auf den Behälter (3) entlang des Umfangs des genanten Halters (6) ist.

11. Die Einrichtung nach Einspruch 9 oder 10, **dadurch gekennzeichnet, dass** die Filtermembran (1) eine Porengröße von 8 pm aufweist.

12. Die Einrichtung nach Einspruch 9 bis 11, **dadurch gekennzeichnet, dass** die Filtermembran (1) aus Polycarbonat hergestellt ist.

13. Die Einrichtung nach Einspruch 9 bis 12, **dadurch gekennzeichnet, dass** das Absorbens (2) aus Zellulose, Feinpapier, Textilfasern oder ihrer Kombination besteht.

14. Verwendung nach Anspruch 7, **dadurch gekennzeichnet dass** für die Durchführung der Detektion und/oder Quantifizierung und/oder Kultivierung die separierten Zellen auf die im Halter (6) nach Anspruch 10 befestigte Filtermembran (1) übertragen werden.

## Revendications

1. Utilisation d'un procédé de séparation de cellules sporadiques présentes dans le liquide organique d'un patient par capture de celles-ci sur une membrane de filtration ayant des dimensions d'orifices inférieures au diamètre des cellules, procédé dans lequel, à partir des cellules sporadiques présentes dans ledit liquide organique présent sur le premier côté de la membrane de filtration, le liquide organique exempt de cellules sporadiques est aspiré à travers la membrane de filtration par la force capillaire dans un matériau absorbant en contact intime avec le second côté de ladite membrane de filtration, aux fins d'obtention desdites cellules sporadiques capturées sur la membrane, qui restent mécaniquement et chimiquement inchangées et donc viables, ce qui permet leur culture efficace ultérieure.

2. Utilisation conformément à la revendication 1, **caractérisée en ce que** les cellules sporadiques présentes dans le liquide organique sont des cellules tumorales en circulation, des cellules tumorales disséminées, des cellules endométriales et des cellules foetales de trophoblaste en circulation.

3. Utilisation conformément à la revendication 1 ou 2, **caractérisée en ce que** le liquide organique est choisi à partir du sang périphérique ou central, de la moelle osseuse, du liquide ascitique, de l'épanchement pleural, du liquide de lavage péritonéal, du liquide de lavage broncho-alvéolaire et du liquide amniotique.

4. Utilisation conformément à n'importe quelle revendication précédente, **caractérisée en ce que** ledit liquide organique est un liquide organique de patients atteints d'un mélanome, d'un cancer du sein, de tumeurs du tractus gastro-intestinal telles que le carcinome gastrique, le carcinome du côlon, le carcinome du pancréas et le carcinome du foie, de tumeurs uro-génitales et de tumeurs des tissus mous tels que les tumeurs de la tête et du cou et d'autres tumeurs solides.

5. Utilisation conformément à la revendication 3, **caractérisée en ce que** le sang périphérique est recueilli sous forme de sang périphérique anticoagulé.

6. Utilisation conformément à n'importe quelle revendication précédente, **caractérisée en ce que,** préalablement à la séparation, le précipité contenant le liquide organique est dilué avec un tampon, en particulier avec du PBS, afin de dissoudre le précipité.

7. Utilisation conformément à n'importe quelle revendication précédente, **caractérisée en ce que,** après la séparation, sont mises en oeuvre la détection et/ou la quantification et/ou la culture des cellules séparées sur la membrane de filtration ou les cellules chassées de la membrane de filtration dans un récipient de culture.

8. Utilisation conformément à la revendication 7, **caractérisée en ce que,** avant la séparation, le liquide organique est dilué avec un milieu de culture, en particulier le RPMI, pour favoriser le succès de la culture ultérieure.

9. Appareil pour la séparation de cellules sporadiques présentes dans le liquide organique d'un patient par capture de celles-ci sur une membrane de filtration ayant des dimensions d'orifices inférieures au diamètre des cellules, procédé dans lequel, à partir des cellules sporadiques présentes dans ledit liquide organique présent sur le premier côté de la membrane de filtration, le liquide organique exempt de cellules sporadiques est aspiré à travers la membrane de filtration par la force capillaire dans un matériau absorbant en contact intime avec le second côté de ladite membrane de filtration, aux fins d'obtention desdites cellules sporadiques capturées sur la membrane, qui restent mécaniquement et chimiquement inchangées et donc viables, ce qui permet leur culture efficace ultérieure, **caractérisé en ce que** ledit appareil comprend un réservoir creux (3) ouvert en haut et en bas pour recevoir un mélange de cellules sporadiques présentes dans le liquide organique, dans lequel la périphérie inférieure du réservoir (3) est en contact étroit avec au moins une partie du premier côté supérieur de la membrane (1), ladite membrane ayant une dimension de pores de 7-10 pm et étant réalisée à partir d'un matériau biocompatible, et dans lequel au moins une partie du deuxième côté inférieur de la membrane (1) est en contact intime avec le matériau absorbant (2), de sorte que la membrane (1) sépare l'espace intérieur du réservoir (3) dudit matériau absorbant (2).

10. Appareil conformément à la revendication 9, **caractérisé en ce que** le matériau absorbant (2) est disposé dans un conteneur de base de collecte (4) pourvu d'un couvercle supérieur (5), dans lequel un support de membrane circonférentiel s'engage de manière amovible à partir du haut, et dans lequel ledit support (6), au moyen d'une bague de pression (7), retient de manière amovible la membrane (1) le long de sa circonférence, et dans lequel le réservoir (3) depuis le support (6) est fermement attaché le long de la circonférence dudit support (6).

11. Appareil conformément aux revendications 9 ou 10, **caractérisé en ce que** la membrane de filtration (1) a une dimension de pores de 8 pm

12. Appareil conformément à n'importe quelle revendication 9 à 11, **caractérisé en ce que** la membrane de filtration (1) est réalisée en polycarbonate.

13. Appareil conformément à n'importe quelle revendication 9 à 12, **caractérisé en ce que** le matériau absorbant (2) est constitué de cellulose, de papier fin, de fibres textiles ou d'une combinaison de ces éléments.

14. Utilisation conformément à la revendication 7, **caractérisée en ce que** pour la réalisation de la détection et/ou de la quantification et/ou de la culture, les cellules séparées sont transférées sur la membrane de filtration (1) fixée dans le support (6) de la revendication 10.
